(19) Europäisches Patentamt
European Patent Office
Office européen des brevets

(11) Publication number: **0 237 341 B1**

(12) **EUROPEAN PATENT SPECIFICATION**

(45) Date of publication of patent specification: **22.09.93**

(51) Int. Cl.[5]: **C07D 493/22**, A61K 31/35, A01N 43/90, //(C07D493/22, 313:00,311:00,311:00,307:00)

(21) Application number: **87302100.0**

(22) Date of filing: **11.03.87**

# (54) Macrolide compounds.

(30) Priority: **12.03.86 GB 8606123**

(43) Date of publication of application:
**16.09.87 Bulletin 87/38**

(45) Publication of the grant of the patent:
**22.09.93 Bulletin 93/38**

(84) Designated Contracting States:
**AT BE CH DE ES FR GB IT LI NL SE**

(56) References cited:
**EP-A- 0 074 758**
**GB-A- 2 176 182**

(73) Proprietor: **AMERICAN CYANAMID COMPANY**
**One Cyanamid Plaza**
**Wayne New Jersey 07470(US)**

(72) Inventor: **Ramsay, Michael V. J.**
**157 Kings Road**
**South Harrow(GB)**
Inventor: **Bell, Richard**
**182 Long Drive**
**South Ruislip(GB)**
Inventor: **Ward, John B.**
**33 Bournehall Road**
**Bushey, Hertfordshire(GB)**
Inventor: **Porter, Neil**
**111 George V Avenue**
**Pinner, Middlesex(GB)**
Inventor: **Noble, Hazel M.**
**113 Stomp Road**
**Burnham, Slough, Buckinghamshire(GB)**
Inventor: **Fletton, Richard A.**
**12 St Edmunds Avenue**
**Ruislip, Middlesex(GB)**
Inventor: **Noble, David**
**113 Stomp Road**
**Burnham, Slough, Buckinghamshire(GB)**
Inventor: **Sutherland, Derek R.**
**41 Milton Fields Chalfont St Giles**
**Buckinghamshire(GB)**

(74) Representative: **Skailes, Humphrey John et al**
**Frank B. Dehn & Co. Imperial House 15-19**
**Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

This invention relates to novel antibiotic compounds and to processes for their preparation.

In our United Kingdom Patent Specification No. 2166436A we describe the production of Antibiotics S541 which may be isolated from the fermentation products of a novel Streptomyces sp. EP-A-074758 describes inter alia 13-desoxy avermectins having a substituted methyl group at the 4-position and a methyl, ethyl, isopropyl or sec-butyl group at the 25-position.

We have now found a further group of compounds with antibiotic activity which may be prepared by chemical modification of Antibiotics S541. The novel compounds of the invention have antibiotic activity and/or are of use as intermediates in the preparation of other active compounds.

Thus, in one aspect, the invention particularly provides the compounds of formula (I)

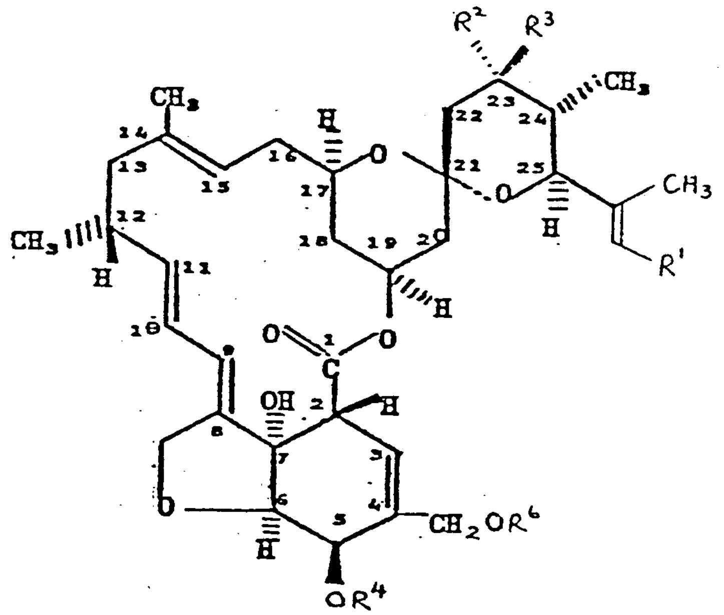

(I)

wherein $R^1$ represents an isopropyl group; $R^2$ represents a hydrogen atom or a group $OR^5$ (where $OR^5$ is a hydroxy group, or a substituted hydroxyl group $>C=NOR^7$ (where $R^7$ represents a hydrogen atom, a $C_{1-8}$ alkyl group or a $C_{3-8}$ alkenyl group, and the group $>C=NOR^7$ is in the E configuration); $OR^4$ represents a group $OR^5$ as defined above; and $R^6$ represents a hydrogen atom or a $C_{1-4}$ alkanoyl group e.g. an acetyl group.

When the compounds of formula (I) are to be used as intermediates, one or both of the groups $R^2$ and $-OR^4$ will often be a protected hydroxy group and the invention particularly includes such protected compounds.

When the groups $R^2$ or $OR^4$ in compounds of formula (I) are substituted hydroxyl groups they may be the same or different and may represent acyloxy groups [e.g. a group of the formula $-OCOR^8$, $-OCO_2R^8$ or $-OCSOR^8$ (where $R^8$ is an aliphatic, araliphatic or aromatic group, for example an alkyl, alkenyl, alkynyl, cycloalkyl, aralkyl or aryl group)], a formyloxy group, a group $-OR^9$ (where $R^9$ is as defined above for $R^8$), a group $-OSO_2R^{10}$ (where $R^{10}$ is a $C_{1-4}$ alkyl or $C_{6-10}$ aryl group), a silyloxy group, a cyclic or acyclic acetaloxy group, a group $OCO(CH_2)_nCO_2R^{11}$ (where $R^{11}$ is a hydrogen atom or a group as defined for $R^8$ above and n represents zero, 1 or 2) or a group $OCONR^{12}R^{13}$ (where $R^{12}$ and $R^{13}$ may each independently represent a hydrogen atom or a $C_{1-4}$ alkyl group eg methyl).

Where $R^8$ or $R^9$ are alkyl groups, they may be for example $C_{1-8}$ alkyl groups e.g. methyl, ethyl, n-propyl, i-propyl, n-butyl, i-butyl, t-butyl or n-heptyl which alkyl groups may also be substituted. Where $R^8$ is a substituted alkyl group it may be substituted by, for example, one or more, e.g. two or three halogen atoms (e.g. chlorine or bromine atoms), or a carboxy, $C_{1-4}$ alkoxy (e.g. methoxy, ethoxy), phenoxy or silyloxy group. Where $R^9$ is a substituted alkyl group it may be substituted by a cycloalkyl e.g. cyclopropyl group.

Where $R^8$ or $R^9$ are alkenyl or alkynyl groups, they may be for example $C_{2-8}$ alkenyl, e.g. allyl, or $C_{2-8}$ alkynyl groups.

Where $R^8$ or $R^9$ are cycloalkyl groups, they may be for example $C_{3-12}$ cycloalkyl, such as $C_{3-7}$ cycloalkyl, e.g. cyclopentyl groups.

Where $R^8$ or $R^9$ are aralkyl groups, they preferably have 1 to 6 carbon atoms in the alkyl moiety and the aryl group(s) may be carbocyclic or heterocyclic and preferably contain 4-15 carbon atoms example a $C_{3-7}$ cycloalkyl, e.g. cyclopentyl group.

Where $R^8$ is an aralkyl group, it may be a phen-$C_{1-6}$ -alkyl, e.g. benzyl, group.

When $R^2$ or $-OR^4$ is a group $-OSO_2R^{10}$, it may be for example a methylsulphonyloxy or p-toluenesulphonyloxy group.

When $R^2$ $-OR^4$ represents a silyloxy group or $R^8$ contains a silyloxy substituent, the silyl group may carry three groups which may be the same or different, selected from alkyl, alkenyl, alkoxy, cycloalkyl, aralkyl, aryl and aryloxy groups. Such groups may be as defined above for $R^8$ and particularly include methyl, t-butyl and phenyl groups. Particular examples of such silyloxy groups are trimethylsilyloxy and t-butyldimethylsilyloxy.

Where $R^2$ or $OR^4$ represents a group $OCO(CH_2)_nCO_2R^{11}$, it may for example be a group $OCOCO_2R^{11}$ or $OCOCH_2CH_2CO_2R^{11}$, where $R^{11}$ represents a hydrogen atom or a $C_{1-4}$ alkyl (e.g. methyl or ethyl) group.

When $R^7$ in the compounds of formula (I) is a $C_{1-8}$ alkyl group, it may be for example a methyl, ethyl, n-propyl, i-propyl, n-butyl or t-butyl group, and is preferably a methyl group. When $R^7$ is a $C_{3-8}$ alkenyl group, it may be for example an allyl group.

The group $R^2$ is preferably a hydrogen atom or a hydroxy group or a group of formula $-OCOR^8$ (where $R^8$ is a $c_{1-8}$ alkyl group optionally substituted by a $C_{1-4}$ alkoxy group), $-OCO_2R^8$ (where $R^8$ is a $C_{1-8}$ alkyl group optionally substituted by one to three halogen atoms e.g. trichloroethyl), $-OCOCO_2H$, $-OR^9$ (where $R^9$ is a $C_{-1-8}$ alkyl, $C_{3-7}$ cyclo-alkyl, allyl or cyclopropylmethyl group), a group $OCONR^{12}R^{13}$ (where $R^{12}$ and $R^{13}$ may each independently represent a hydrogen atom or a methyl group) or $R^2$ and $R^3$ together with the carbon atom to which they are attached form a $>C=O$, $>C=CH_2$ or $>C=NOCH_3$ group. In particular, $R^2$ is preferably a hydrogen atom or an ethoxy, n-propoxy, cyclopropylmethoxy, acetoxy, propionoxy, isobutyrionoxy or cyclopropanecarbonyloxy group or $R^2$ and $R^3$ together with the carbon atom to which they are attached form a $>C=O$, $>C=CH_2$ or $>C=NOCH_3$ group.

The group $-OR^4$ in the compounds of formula (I) is preferably a hydroxy, methoxy, acetoxy or methyloxycarbonyloxy group.

Important active compounds according to the invention are those of formula (I) in which $R^1$ is an isopropyl group, $R^2$ is a hydroxyl or acetoxy group, $R^3$ is a hydrogen atom, $OR^4$ is a hydroxy or acetoxy group and $R^6$ is a hydrogen atom or an acetyl group.

A particularly important active compound of the invention is that of formula (I) in which:

$R^1$ is an isopropyl group, $R^2$ and $R^3$ are hydrogen atoms, $OR^4$ is an acetoxy group and $R^6$ is a hydrogen atom.

As indicated previously, the compounds according to the invention may be of use as antibiotics and/or as intermediates for the preparation of other active compounds. When the compounds of the invention are to be used as intermediates, the $R^2$ and/or $-OR^4$ groups may be protected hydroxyl groups. It will be appreciated that such a group should have the minimum of additional functionality to avoid further sites of reaction and should be such that it is possible to selectively regenerate a hydroxyl group from it. Examples of protected hydroxyl groups are well known end are described, for example, in "Protective Groups in Organic Synthesis" by Theodora W. Greene. (Wiley-Interscience, New York 1981) and "Protective Groups in Organic Chemistry" by J F W McOmie (Plenum Press, London, 1973). Examples of $R^2$ and $OR^4$ protected hydroxy groups include phenoxyacetoxy, silyloxyacetoxy, (e.g. trimethylsilyloxyacetoxy and t-butyldimethylsilyloxyacetoxy), and silyloxy such as trimethylsilyloxy and t-butyldimethylsilyloxy. Compounds of the invention containing such groups will primarily be of use as intermediates. Other groups, such as acetoxy, may serve as protected hydroxyl groups, but may also be present in final active compounds.

Other active compounds of the invention that are also useful as intermediates are those of formula (I) in which $R^2$ is a $-OCOCO_2H$ group.

Compounds of the invention have antibiotic activity e.g. antihelminthic activity, for example against nematodes, and in particular, anti-endoparasitic and anti-ectoparasitic activity.

The compounds of the invention are therefore of use in treating animals and humans with endoparasitic and/or ectoparasitic infections.

Ectoparasites and endoparasites infect humans and a variety of animals and are particularly prevalent in farm animals such as pigs, sheep, cattle, goats and poultry (e.g. chickens and turkeys), horses, rabbits, game-birds, caged birds, and domestic animals such as dogs, cats, guinea pigs, gerbils and hamsters. Parasitic infection of livestock, leading to anaemia, malnutrition and weight loss is a major cause of

economic loss throughout the world.

Examples of genera of endoparasites infecting such animals and/or humans are Ancylostoma, Ascaridia, Ascaris, Aspicularis, Brugia, Bunostomum, Capillaria, Chabertia, Cooperia, Dictyocaulus, Dirofilaria, Dracunculus, Enterobius, Haemonchus, Heterakis, Loa, Necator, Nematodirus, Nematospiroides (Heligomoroides), Nippostrongylus, Oesophagostomum, Onchocerca, Ostertagia, Oxyuris, Parascaris, Strongylus, Strongyloides, Syphacia, Toxascaris, Toxocara, Trichonema, Trichostrongylus, Trichinella, Trichuris, Triodontophorus, Uncinaria and Wuchereria.

Examples of ectoparasites infecting animals and/or humans are arthropod ectoparasites such as biting insects, blowfly, fleas, lice, mites, sucking insects, ticks and other dipterous pests.

Examples of genera of such ectoparasites infecting animals and/or humans are Ambylomma, Boophilus, Chorioptes, Culliphore, Demodex, Damalinia, Dermatobia, Gastrophilus, Haematobia, Haematopinus, Haemophysalis, Hyaloma, Hypoderma, Ixodes, Linognathus, Lucilia, Melophagus, Oestrus, Otobius, Otodectes, Psorergates, Psoroptes, Rhipicephalus, Sarcoptes, Stomoxys and Tabanus.

The compounds according to the invention have been found to be effective both in vitro and in vivo against a range of endoparasites and ectoparasites. The antibiotic activity of compounds of the invention may, for example, be demonstrated by their activity against free living nematodes e.g. Caenorhabiditis elegans. In particular, we have found that compounds of the invention are active in vivo against parasitic nematodes such as Nematospiroides dubius.

Compounds of the invention are also of use as anti- fungals, for example, against strains of Candida sp. such as Candida albicans and Candida glabrata and against yeast such as Saccharomyces carlsbergensis.

Compounds of the invention are also of use in combating insect, acarine and nematode pests in agriculture, horticulture, forestry, public health and stored products. Pests of soil and plant crops, including cereals (e.g. wheat, barley, maize and rice), cotton, tobacco, vegetables (e.g. soya), fruit (e.g. apples, vines and citrus) as well as root crops (e.g. sugarbeet, potatoes) may usefully be treated. Particular examples of such pests are fruit mites and aphids such as Aphis fabae, Aulacorthum circumflexum, Myzus persicae, Nephotettix cincticeps, Nilparvata lugens, Panonychus ulmi, Phorodon humuli, Phyllocoptruta oleivora, Tetranychus urticae and members of the genera Trialeuroides; nematodes such as members of the genera Aphelencoides, Globodera, Heterodera, Meloidogyne and Panagrellus; lepidoptera such as Heliothis, Plutella and Spodoptera; grain weevils such as Anthonomus grandis and Sitophilus granarius; flour beetles such as Tribolium castaneum; flies such as Musca domestica; fire ants; leaf miners; Pear psylla; Thrips tabaci; cockroaches such as Blatella germanica and Periplaneta americana and mosquitoes such as Aedes aegypti.

According to the invention we therefore provide compounds of formula (I) as defined above, which may be used as antibiotics. In particular, they may be used in the treatment of animals and humans with endoparasitic, ectoparasitic and/or fungal infections and in agriculture, horticulture, or forestry as pesticides to combat insect, acarine and nematode pests. They may also be used generally as pesticides to combat or control pests in other circumstances, e.g. in stores, buildings or other public places or location of the pests. In general the compounds may be applied either to the host (animal or human or plants or vegetation) or a locus thereof or to the pests themselves.

Compounds of the invention may be formulated for administration in any convenient way for use in veterinary or human medicine and the invention therefore includes within its scope pharmaceutical compositions comprising a compound in accordance with the invention adapted for use in veterinary or human medicine. Such compositions may be presented for use in conventional manner with the aid of one or more suitable carriers or excipients. The compositions of the invention include those in a form especially formulated for parenteral (including intramammary administration), oral, rectal, topical, implant, ophthalmic, nasal or genito-urinary use.

The compounds according to the invention may be formulated for use in veterinary or human medicine by injection and may be presented in unit dose form, in ampoules, or other unit-dose containers, or in multi-dose containers, if necessary with an added preservative, The compositions for injection may be in the form of suspensions, solutions, or emulsions, in oily or aqueous vehicles, and may contain formulatory agents such as suspending, stabilising, solubilising and/or dispersing agents. Alternatively the active ingredient may be in sterile powder form for reconstitution with a suitable vehicle, e.g. sterile, pyrogen-free water, before use. Oily vehicles include polyhydric alcohols and their esters such as glycerol esters, fatty acids, vegetable oils such as arachis oil or cottonseed oil, mineral oils such as liquid paraffin, and ethyl oleate and other similar compounds. Other vehicles such as propylene glycol may also be used.

Compositions for veterinary medicine may also be formulated as intramammary preparations in either long acting or quick-release bases and may be sterile solutions or suspensions in aqueous or oily vehicles optionally containing a thickening or suspending agent such as soft or hard paraffins, beeswax, 12-hydroxy stearin, hydrogenated castor oil, aluminium stearates, or glyceryl monostearate. Conventional non-ionic,

cationic or anionic surface active agents may be used alone or in combination in the composition.

The compounds of the invention may also be presented for veterinary or human use in a form suitable for oral administration, for example in the form of solutions, syrups or suspensions, or a dry powder for constitution with water or other suitable vehicle before use, optionally with flavouring and colouring agents. Solid compositions such as tablets, capsules, lozenges, pills, boluses, powder, pastes, granules, bullets or premix preparations may also be used. Solid and liquid compositions for oral use may be prepared according to methods well known in the art. Such compositions may also contain one or more pharmaceutically acceptable carriers and excipients which may be in solid or liquid form. Examples of suitable pharmaceutically acceptable carriers for use in solid dosage forms include binding agents (e.g. pregelatinised maize starch, polyvinylpyrrolidone or hydroxypropyl methylcellulose); fillers (e.g. lactose, micro-crystalline cellulose or calcium phosphate); lubricants (e.g. magnesium stearate, talc or silica); disintegrants (e.g. potato starch or sodium starch glycollate); or wetting agents (e.g. sodium lauryl sulphate). Tablets may be coated by methods well known in the art.

Examples of suitable pharmaceutically acceptable additives for use in liquid dosage forms include suspending agents (e.g. sorbitol syrup, methyl cellulose or hydrogenated edible fats); emulsifying agents (e.g. lecithin or acacia); non-aqueous vehicles (e.g. almond oil, oily esters or ethyl alcohol); and preservatives (e.g. methyl or propyl p-hydroxybenzoates or sorbic acid); stabilising and solubilising agents may also be includeed.

Pastes for oral administration may be formulated according to methods well known in the art. Examples of suitable pharmaceutically acceptable additives for use in paste formulations include suspending or gelling agents e.g. aluminium distearate or hydrogenated castor oil; dispersing agents e.g. polysorbates, non-aqueous vehicles e.g. arachis oil or oily esters; stabilising and solubilising agents. The compounds of the invention may also be administered in veterinary medicine by incorporation thereof into animals daily solid or liquid dietary intake, e.g. as part of the daily animal feed or drinking water.

The compounds of the invention may also be administered orally in veterinary medicine in the form of a liquid drench such as a solution, suspension or dispersion of the active ingredient together with a pharmaceutically acceptable carrier or excipient.

The compounds of the invention may also, for example, be formulated as suppositories e.g. containing conventional suppository bases for use in veterinary or human medicine or as pessaries e.g. containing conventional pessary bases.

Compounds according to the invention may be formulated for topical administration, for use in veterinary and human medicine, as ointments, creams, lotions, shampoos, powders, pessaries, sprays, dips, aerosols, drops (e.g. eye or nose drops) or pour-ons. Ointments and creams may, for example, be formulated with an aqueous or oily base with the addition of suitable thickening and/or gelling agents. Ointments for administration to the eye may be manufactured in a sterile manner using sterilised components. Pour-ons may, for example, be formulated for veterinary use in oils containing organic solvents, optionally with formulatory agents e.g. stabilising and solubilising agents.

Lotions may be formulated with an aqueous or oily base and will in general also contain one or more emulsifying agents, stabilising agents, dispersing agents, suspending agents, thickening agents, or colouring agents.

Powders may be formed with the aid of any suitable powder base. Drops may be formulated with an aqueous or non aqueous base also comprising one or more dispersing agents, stabilising agents, solubilising agents or suspending agents. They may also contain a preservative.

For topical administration by inhalation the compounds according to the invention may be delivered for use in veterinary or human medicine in the form of an aerosol spray presentation or an insufflator.

The compounds of the invention may be administered in combination with other pharmaceutically active ingredients.

The total daily dosages of compounds of the invention employed in both veterinary and human medicine will suitably be in the range 1-2000$\mu$g/kg bodyweight, preferably from 50-1000$\mu$g/kg and these may be given in divided doses, e.g. 1-4 times per day.

The compounds according to the invention may be formulated in any convenient way for horticultural or agricultural use and the invention therefore includes within its scope compositions comprising a compound according to the invention adapted for horticultural or agricultural use. Such formulations include dry or liquid types, for example dusts, including dust bases or concentrates, powders, including soluble or wettable powders, granulates, including microgranules and dispersible granules, pellets, flowables, emulsions such as dilute emulsions or emulsifiable concentrates, dips such as root dips and seed dips, seed dressings, seed pellets, oil concentrates, oil solutions, injections e.g. stem injections, sprays, smokes and mists.

Generally such formulations will include the compound in association with a suitable carrier or diluent. Such carriers may be liquid or solid and designed to aid the application of the compound either by way of dispersing it where it is to be applied or to provide a formulation which can be made by the user into a dispersible preparation. Such formulations are well known in the art and may be prepared by conventional methods such as, for example by blending and/or grinding of the active ingredient(s) together with the carrier or diluent, e.g. solid carrier, solvent or surface active agent.

Suitable solid carriers, for use in the formulations such as dusts, granulates and powders may be selected from for example natural mineral fillers, such as diatomite, talc, kaolinite, montmorillonite prophyllite or attapulgite. Highly dispersed silicic acid or highly dispersed absorbent polymers may, if desired, be included in the composition. Granulated adsorptive carriers which may be used may be porous (such as pumice, ground brick, sepiolite or bentonite) or no-porous (such as calcite or sand). Suitable pregranulated materials which may be used and which may be organic or inorganic include dolomite and ground plant residues.

Suitable solvents for use as carriers or diluents include aromatic hydrocarbons, aliphatic hydrocarbons, alcohols and glycols or ethers thereof, esters, ketones, acid amides, strongly polar solvents, optionally epoxidized vegetable oils and water.

Conventional non-ionic, cationic or anionic surface-active agents, e.g. ethoxylated alkyl phenols and alcohols, alkali metal or alkaline earth metal salts of alkyl benzene sulphonic acids, lignosulphonic acids or sulphosuccinic acids or sulphonates of polymeric phenols which have good emulsifying, dispersing and/or wetting properties may also be used either alone or in combination in the compositions.

Stabilizers, anti-caking agents, anti-foaming agents, viscosity regulators, binders and adhesives, photostabilisers as well as fertilizers, feeding stimulants or other active substances may, if desired, be included in the compositions. The compounds of the invention may also be formulated in admixture with other insecticides, acaricides and nematicides.

In the formulations, the concentration of active material is generally from 0.01 to 99% and more preferably between 0.01% and 40% by weight.

Commercial products are generally provided as concentrated compositions to be diluted to an appropriate concentration, for example from 0.001 to 0.0001% by weight, for use.

The compounds of the invention may be prepared by the processes discussed below. In some of these processes it may be necessary to protect a hydroxyl group at the 5- or 23-position in the starting material prior to effecting the reaction described. In such cases it may then be necessary to deprotect the same hydroxyl group once the reaction has occurred to obtain the desired compound of the invention. Conventional protection and deprotection methods may be used, for example as described in the aforementioned books by Greene and McOmie.

Thus, for example, an acyl group such as an acetyl group may be removed by basic hydrolysis e.g. using sodium or potassium hydroxide in aqueous alcohol. Acetal groups such as tetrahydropyranyl may be removed for example, using acid hydrolysis (using an acid such as acetic or trifluoroacetic acid or a dilute mineral acid). Silyl groups may be removed using fluoride ions (e.g. from a tetraalkylammonium fluoride such as tetra-n-butylammonium fluoride), hydrogen fluoride in aqueous acetonitrile or an acid such as p-toluene sulphonic acid (e.g. in methanol). Arylmethyl groups may be removed by treatment with a Lewis acid (e.g. boron trifluoride-etherate) in the the presence of a thiol (e.g. ethanethiol) in a suitable solvent such as dichloromethane at e.g. room temperature. Selective 5-deprotection of a 5,23-disilyl compound of the invention may be effected using tetra-n-butylammonium fluoride while selective deprotection of a 5,23-diacetoxy compound may be effected using sodium hydroxide in aqueous methanol.

According to a further aspect of the invention we provide a process for the preparation of compounds of formula (I) in which $R^6$ represents a hydrogen atom which comprises reacting a compound of formula (II):

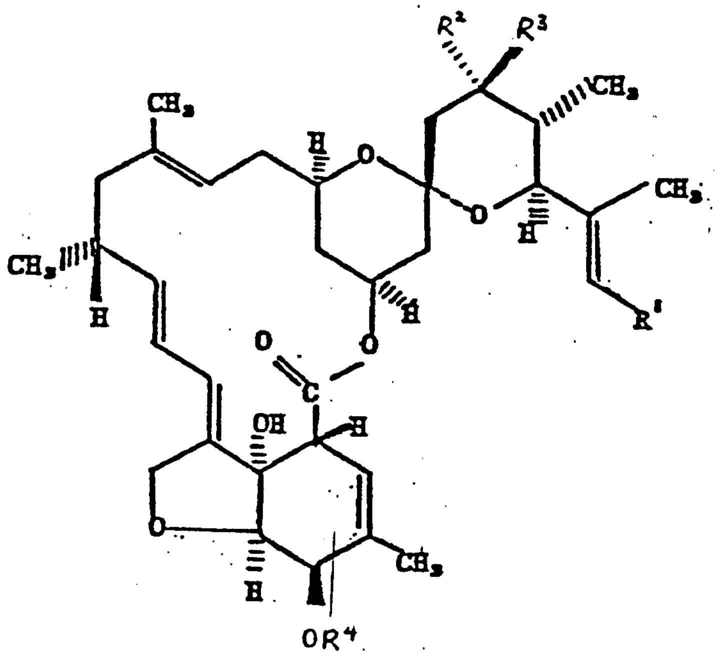

(II)

(where $R^1$-$R^4$ are as previously defined) with an oxidising agent serving to convert the 4-methyl group into a 4-hydroxymethyl group without affecting any other groups in the molecule susceptible to oxidation.

A suitable oxidising agent for the conversion is selenium dioxide, preferably in the presence of an activator such as tert-butyl hydroperoxide. The reaction may conveniently be effected in an inert solvent such as a halogenated hydrocarbon e.g. dichloromethane, ethyl acetate or an ether e.g. tetrahydrofuran, at a temperature in the range of 0 to 50 °C, preferably at room temperature.

Compounds of formula (I) in which $R^6$ represents a $C_{1-4}$ alkanoyl group may be prepared from a corresponding compound of formula (I) in which $R^6$ represents -H . The acylation reaction may be carried out using conditions which will selectively acylate the primary alcohol but leave any remaining secondary and tertiary hydroxyl groups unaffected. The reaction may thus be carried out using an acid $R^6OH$ (where $R^6$ is a $C_{1-4}$ alkanoyl group) in the presence of a triarylphosphine e.g. triphenylphosphine and a dialkyl diazodicarboxylate in an inert solvent such as a hydrocarbon e.g. benzene or toluene, an ether e.g. tetrahydrofuran or an ester e.g. ethyl acetate at a temperature of from -15 to 80 °C.

Compounds of formula (I) in which one of $R^2$ and $OR^4$ is a substituted hydroxyl group and the other is a hydroxyl or substituted hydroxyl group as defined above and $R^6$ is a $C_{1-4}$ alkanoyl group may be prepared by reacting compounds of formula (III):-

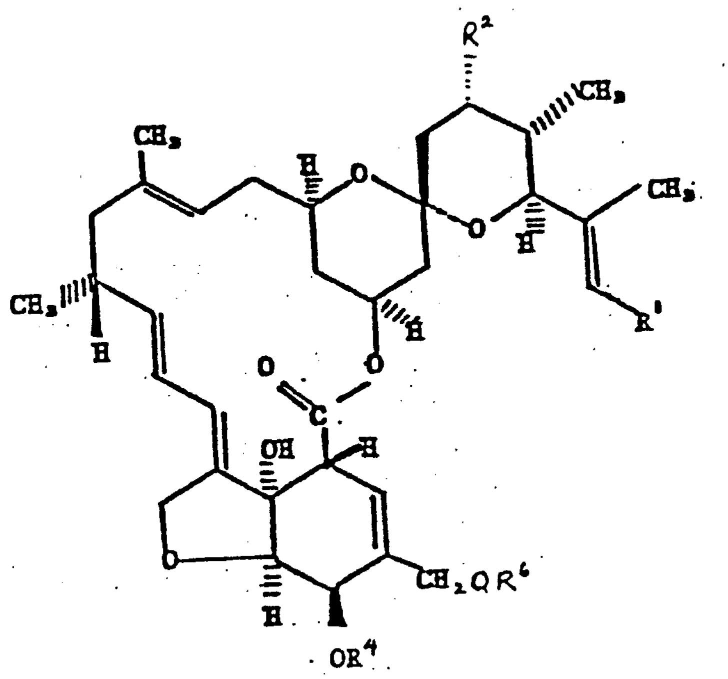

(III)

(where $R^1$ is as previously defined, one of $R^2$ and $OR^4$ is a hydroxyl group while the other is a hydroxyl or substituted hydroxyl group and $R^6$ is a hydrogen atom or a $C_{1-4}$ alkanoyl group) with a reagent serving to convert a hydroxyl group into a substituted hydroxyl group, and if desired followed by selective deprotection of a compound of formula (I) in which $R^2$ and $-OR^4$ are both substituted hydroxyl groups to give a compound of formula (I) in which $OR^4$ is a hydroxyl group and $R^2$ is a substituted hydroxyl group.

The reaction will in general be an acylation, formylation, sulphonylation, etherification, silylation or acetal formation.

Thus, for example, acylation may be effected using an acylating agent such as an acid of formula $R^8COOH$ or a reactive derivative thereof, such as an acid halide (e.g. acid chloride), anhydride or activated ester, or a reactive derivative of a carbonic acid $R^8OCOOH$ or thiocarbonic acid $R^8OCSOH$.

Acylations employing acid halides and anhydrides may if desired be effected in the presence of an acid binding agent such as a tertiary amine (e.g. triethylamine, dimethylaniline or pyridine), inorganic bases (e.g. calcium carbonate or sodium bicarbonate), and oxiranes such as lower 1,2-alkylene oxides (e.g. ethylene oxide or propylene oxide) which bind hydrogen halide liberated in the acylation reaction.

Acylations employing acids are desirably conducted in the presence of a condensing agent, for example a carbodiimide such as N,N'-dicyclohexylcarbodiimide or N-ethyl-N'γ-dimethylaminopropylcarbodiimide; a carbonyl compound such as carbonyldiimidazole; or an isoxazolium salt such as N-ethyl-5-phenylisoxazolium perchlorate.

An activated ester may conveniently be formed in situ using, for example, 1-hydroxybenzotriazole in the presence of a condensing agent as set out above. Alternatively, the activated ester may be preformed.

The acylation reaction may be effected in aqueous or non-aqueous reaction media, conveniently at a temperature in the range -20° to +100°C, e.g. -10° to +50°C.

Formylation may be effected using an activated derivative of formic acid e.g. N-formyl imidazole or formic acetic anhydride under standard reaction conditions.

Sulphonylation may be effected with a reactive derivative of a sulphonic acid $R^{10}SO_3H$ such as a sulphonyl halide, for example a chloride $R^{10}SO_2Cl$. The sulphonylation is preferably effected in the presence of a suitable acid binding agent as described above.

Etherfication may be effected using a reagent of formula $R^9Y$ (where $R^9$ is as previously defined and Y represents a leaving group such as chlorine, bromine or iodine atom or a hydrocarbylsulphonyloxy group, such as mesyloxy or tosyloxy, or a haloalkanoyloxy group such as dichloroacetoxy). The reaction may be carried out by formation of a magnesium alkoxide using a Grignard reagent such as a methylmagnesium halide e.g. methylmagnesium iodide or using a trialkylsilylmethylmagnesium halide e.g. trimethylsilylmethylmagnesium chloride followed by treatment with the reagent $R^9Y$.

Alternatively, the reaction may be effected in the presence of a silver salt such as silver oxide, silver perchlorate, silver carbonate or silver salicylate or mixtures thereof, and this system may be particularly

appropriate when etherification is carried out using an alkyl halide (e.g. methyl iodide).

Etherification may conveniently be effected in a solvent such as an ether e.g. diethyl ether.

Acetal formation may be carried out by reaction with a cyclic or acyclic vinyl ether. This method is especially useful for production of tetrahydropyranyl ethers, using dihydropyran as reagent, or 1-alkoxyalkyl ethers such as 1-ethoxyalkyl ether, using an alkyl vinyl ether as reagent. The reaction is desirably carried out in the presence of a strong acid catalyst, for example a mineral acid such as sulphuric acid, or an organic sulphonic acid such as p-toluene sulphonic acid, in a non-hydroxylic, substantially water-free solvent.

Silylation may be effected by reaction with a silyl halide (e.g. chloride), advantageously in the presence of a base such as imidazole, triethylamine or pyridine, using a solvent such as dimethylformamide.

Compounds of formula (II) in which $R^2$ represents a hydrogen atom or a group $OR^5$ and $R^3$ is hydrogen atom, or $R^2$ and $R^3$ together with the carbon atom to which they are attached represent $>C=O$ are either known compounds described in UK Patent Specification No. 2176182A or may be prepared from the known compounds using methods analogues to those described therein.

Compounds of formula (II) in which $R^2$ and $R^3$ together with the carbon atom to which they are attached represent $>C=CH_2$ may be prepared by reacting the corresponding known 23-keto compounds (i.e. compounds of formula (II) in which $R^2$ and $R^3$ together with the carbon atom to which they are attached represent $>C=O$) with an appropriate Wittig reagent e.g. a phosphorane of formula $(R^a)_3P=CH_2$ (where $R^a$ represents $C_{1-6}$ alkyl or aryl, e.g. monocyclic aryl such as phenyl). Suitable reaction solvents include ethers such as tetrahydrofuran or diethyl ether or a dipolar aprotic solvent such as dimethyl sulphoxide. The reaction may be carried out at any suitable temperature e.g. at 0 °C.

Compounds of formula (II) in which $R^2$ and $R^3$ together with the carbon atom to which they are attached represent $>C=NOR^7$ [where $R^7$ is as defined in formula (I)] may be prepared from the corresponding 23-keto compounds by reaction with a reagent $H_2NOR^7$ (where $R^7$ is as just defined).

The reaction may conveniently be effected at a temperature in the range -20 to +100 °C, e.g. -10 to +50 °C. It is convenient to use the reagent $H_2NOR$ in the form of a salt, for example an acid addition salt such as the hydrochloride. When such a salt is employed the reaction may be carried out in the presence of an acid binding agent.

Solvents which may be employed include alcohols (e.g. methanol or ethanol), amides (e.g. N,N-dimethylformamide, N,N-dimethylacetamide or hexamethylphosphoramide), ethers (e.g. cyclic ethers such as tetrahydrofuran or dioxan, and acylic ethers such as dimethoxyethane or diethyl ether), nitriles (e.g. acetonitrile), sulphones (e.g. sulpholane) and hydrocarbons such as halogenated hydrocarbons (e.g. methylene chloride), as well as mixtures of two or more such solvents. Water may also be employed as a cosolvent.

When aqeuous conditions are employed the reaction may conveniently be buffered with an appropriate acid, base or buffer.

Suitable acids include mineral acids, such as hydrochloric or sulphuric acid, and carboxylic acid such as acetic acid. Suitable bases include alkali metal carbonates and bicarbonates such as sodium bicarbonate, hydroxides such as sodium hydroxide, and alkali metal carboxylates such as sodium acetate. A suitable buffer is sodium acetate/acetic acid.

The invention is further illustrated by the following Examples. All temperatures are in °C. The compounds are hereinafter named by reference to the known parent "Factor", Factor A, which is a compound of formula (II) in which $R^1$ is isopropyl, $R^2$ is hydroxy, $R^3$ is hydrogen and $R^4$ is hydrogen. Factor A may be prepared as described in UK Patent Specification No. 2166436A.

Example 1

4a-Hydroxy Factor A

Selenium dioxide (83 mg) was added to a solution of t-butylhydroperoxide (3M in dichloromethane; 1 ml) in dichloromethane (5 ml). After stirring at room temperature for 30 min, Factor A (919mg) was added and stirring was continued for a further 24 h. The reaction mixture was diluted with ethyl acetate (100 ml), washed with water and brine, and dried ($Na_2SO_4$). The solvent was evaporated and the residue purified by flash chromatography (35 g silica gel, Merck 9385). Elution with ethyl acetate afforded the title compound as a pale yellow foam (215 mg), $[\alpha]_D^{22}$ + 136° (c 0.92, $CHCl_3$); $\lambda_{max}$ (EtOH) 243.8 nm ($\epsilon$ 28,500); $\nu_{max}$ ($CHBr_3$) 3510 (OH), 1710 (esters), 997 $cm^{-1}$ (C-O); $\delta$ ($CDCl_3$) values include 4.58 (1H,m),4.33 (1H, d, J 14Hz,), 4.24 (1H, d, J 14Hz), 4.58 (1H,m), 3.82 (1H,m),3.75 (1H,d, J 10Hz), 0.79 (3H, d, J 7Hz).

Example 2

4a-Hydroxy-23-desoxy Factor A, 5-acetate

23-Desoxy Factor A, 5-acetate (4.791g, Example 112 in UK 2176182A) was added to a stirred mixture of selenium dioxide (416mg) and t-butyl hydroperoxide (3M in dichloromethane; 5ml) in dichlorormethane (30ml). After stirring at room temperature for 30h the reaction mixture was diluted with ethyl acetate (200 ml), washed with water and brine, and dried ($Na_2SO_4$). The solvent was evaporated and the residue purified by flash chromatography (250g silica gel, Merck 9385). Elution with ethyl acetate: light petroleum (1:4→1:2) afforded a number of fractions including the title compound (292 mg), $\lambda_{max}$ (EtOH) 243.6 nm ($\epsilon$ 29,100); $\nu_{max}$($CHBr_3$) 3600, 3480 (OH), 1732 (OAc), 1710 ($CO_2R$), 996cm$^{-1}$ (C-O); $\delta$ ($CDCl_3$) values include 0.69 (3H, d, J 5 Hz), 2.16 (3H,s), 3.36 (1H,m), 4.10 (1H, d, J 6 Hz), 4.13 (2H, d, J 6 Hz), 5.90 (1H,s).

In a similar manner were prepared Examples 3 and 4.

Example 3

4a-Hydroxy-23-desoxy Factor A

23-Desoxy Factor A (597 mg, Example 27 in UK 2176182A), after flash chromatography (40 g silica gel, Merck 9385) eluting with ethyl acetate : light petroleum (1:1 → 1:0), gave the title compound as a yellow foam (131 mg), $\lambda_{max}$ (EtOH) 243.6 nm ($\epsilon$24,900); $\nu_{max}$ ($CHBr_3$) 3560, 3500 (OH), 1708cm$^{-1}$ ($CO_2R$); $\delta$ - ($CDCl_3$) values include 0.69 (3H, d, J4Hz), 0.94 (3H, d, J6Hz), 1.00 (3H, d, J6Hz), 1.04 (3H, d, J6Hz), 3.32 (1H, m), 3.97 (1H, d, J5Hz), 4.2-4.4 (2H, m), 4.57 (1H, d, J5Hz).

Example 4

4a-Hydroxy Factor A, 5-t-butyldimethylsilyl ether

Factor A, 5-t-butyldimethylsilyl ether (2.181g, Example 6 in UK 2176182A), after flash chromatography (200 g silica gel, Merck 9385) eluting with ethyl acetate : light petroleum (1:4 → 1:2) afforded the title compound as a yellow foam (450 mg), $\nu_{max}$ ($CHBr_3$) 3510 (OH), 1710cm$^{-1}$ ($CO_2R$); $\delta$ ($CDCl_3$) values include 0.14 (6H, s), 0.79 (3H, d, J6Hz), 0.92 (9H, s), 0.94 (3H, d, J6Hz), 0.98 (3H, d, J6Hz), 1.04 (3H, d, J6Hz), 3.38 (1H, m), 3.79 (1H, d, J5Hz), 4.0-4.3 (2H, m).

Example 5

4a-Hydroxy Factor A 4a,5,23-triacetate

Acetic anhydride (78 µl) was added to a stirred solution of 4a-hydroxy Factor A (52 mg) in dry pyridine (1 ml), under an atmosphere of nitrogen. After 30 min at room temperature 4-dimethylaminopyridine (1 mg) was added and stirring continued for a further 24 h. The reaction mixture was diluted with ethyl acetate (50 ml), washed with 2M hydrochloric acid, saturated sodium bicarbonate solution and brine, and dried ($Na_2SO_4$). The solvent was evaporated and the residue purified by chromatography (20 g silica gel, Merck 9385). Elution with ethyl acetate:light petroleum (1:2) afforded the title compound as a white foam (40 mg); $[\alpha]_D^{22}$ + 148° (c 0.89, $CHCl_3$); $\lambda_{max}$ (EtOH) 244 nm ($\epsilon$ 31,000); $\nu_{max}$ ($CHBr_3$) 3470 (OH), 1730 (esters), 997 cm$^{-1}$ (C-O); $\delta$ ($CDCl_3$) values include 5.91 (1H,s), 4.92 (1H,d,J,10Hz),4.91(1H,m), 4.4-4.8 (4H,m), 2.14 (3H,s), 2.06 (3H,s), 2.04 (3H,s), 0.71 (3H,d,J 7Hz); [Found : C,66.5; H,7.9. $C_{42}H_{58}O_{12}$ requires C,66.8; H,7.75%]. Further elution gave 4a-hydroxy Factor A 4a,5-diacetate as a white foam (8 mg,); $\nu_{max}$ ($CHBr_3$) 3500 (OH), 1736 (broad, esters), 995 cm$^{-1}$ (C-O); $\delta$ ($CDCl_3$) values include 5.91 (1H,s), 4.4-4.8 (4H,m), 3.82 (1H,m), 3.75 (1H,d,J 10Hz), 2.14 (3H,s), 2.07 (3H,s), 0.70 (3n,d,J 7Hz).

Example 6

4a-Acetoxy-23-desoxy Factor A, 5-acetate

A solution of 4a-hydroxy-23-desoxy Factor A, 5-acetate (100mg), in dry pyridine (1.5 ml) was treated with acetic anhydride (72µl) under an atmosphere of nitrogen. After stirring at room temperature for 20h the reaction mixture was diluted with ethyl acetate (50ml), washed with 2M hydrochloric acid, saturated sodium

bicarbonate solution and brine and dried ($Na_2SO_4$). The solvent was evaporated and the residue purified by flash chromatography (20g silica gel, Merck 9385). Elution with ethyl acetate : light petroleum (1:3) afforded the title compound as a pale yellow foam (73mg); $\lambda_{max}$ (EtOH) 244.4nm ($\epsilon$30,000); $\nu_{max}$ ($CHBr_3$) 3460 (OH), 1732 (OAc), 1710cm$^{-1}$ ($CO_2R$); $\delta$ ($CDCl_3$) values include 0.69 (3H, d, J5Hz), 0.94 (3H, d, J6Hz), 1.00 (3H, d, J6Hz), 1.04 (3H, d, J6Hz), 2.05 (3H, s), 2.13 (3H, s), 3.36 (1H, m), 4.10 (1H, d, J5Hz), 4.4-4.8 (4H, m).

Example 7

4a-Acetoxy-23-desoxy Factor A

Diethyl azodicarboxylate (28µl) was added to a stirred solution of 4a-hydroxy-23-desoxy Factor A (90mg), triphenylphosphine (47mg) and acetic acid (10µl) in toluene (2ml), under an atmosphere of nitrogen. After 2h at room temperature the reaction mixture was evaporated and the residue purified by flash chromatography (20g silica gel, Merck 9385). Elution with ethyl acetate : hexane (1:1) gave the title compound as a white foam (37 mg); $\lambda_{max}$ (EtOH) 244.2nm ($\epsilon$29,900); $\nu_{max}$ ($CHBr_3$) 3540, 3480 (OH), 1738 (OAc), 1710cm$^{-1}$ ($CO_2R$); $\delta$ ($CDCl_3$) values include 0.69 (3H, d, J5Hz), 0.93 (3H, d, J6Hz), 0.99 (3H, d, J6Hz), 1.03 (3H, d, J6Hz), 2.09 (3H, s), 3.32 (1H, m), 4.48 (1H, t, J5Hz).

The following are examples of formulations according to the invention. The term 'Active Ingredient' as used hereinafter means a compound of the invention.

Multidose parenteral injection

| | % w/v | Range |
|---|---|---|
| Active Ingredient | 4.0 | 0.1 - 7.5% w/v |
| Benzyl alcohol | 2.0 | |
| Glyceryl triacetate | 30.0 | |
| Propylene glycol | to 100.0 | |

Dissolve the active ingredient in the benzyl alcohol and glyceryl triacetate. Add propylene glycol and make up to volume. Sterilise the product by conventional pharmaceutical methods, for example sterile filtration or by heating in an autoclave and package aseptically.

Aerosol spray

| | % w/w | Range |
|---|---|---|
| Active Ingredient | 0.1 | 0.01 - 2.0% w/w |
| Trichloroethane | 29.9 | |
| Trichlorofluoromethane | 35.0 | |
| Dichlorodifluoromethane | 35.0 | |

Mix the Active Ingredient with trichloroethane and fill into the aerosol container. Purge the headspace with the gaseous propellant and crimp the valve into position. Fill the required weight of liquid propellant under pressure through the valve. Fit with actuators and dust-caps.

Tablet

Method of manufacture - wet granulation

| | mg |
|---|---|
| Active Ingredient | 250.0 |
| Magnesium stearate | 4.5 |
| Maize starch | 22.5 |
| Sodium starch glycolate | 9.0 |
| Sodium lauryl sulphate | 4.5 |
| Microcrystalline cellulose | to tablet core weight of 450mg |

Add sufficient quantity of a 10% starch paste to the active ingredient to produce a suitable wet mass for granulation. Prepare the granules and dry using a tray or fluid-bed drier. Sift through a seive, add the remaining ingredients and compress into tablets.

If required, film coat the tablet cores using hydroxypropylmethyl cellulose or other similar film-forming material using either an aqueous or non-aqueous solvent system. A plasticizer and suitable colour may be included in the film-coating solution.

Veterinary tablet for small/domestic animal use

Method of manufacture - dry granulation

| | mg |
|---|---|
| Active Ingredient | 50.0 |
| Magnesium stearate | 7.5 |
| Microcrystalline cellulose to tablet core weight of | 75.0 |

Blend the active ingredient with the magnesium stearate and microcrystallise cellulose. Compact the blend into slugs. Break down the slugs by passing through a rotary granulator to produce free-flowing granules. Compress into tablets.

The tablet cores can then be film-coated, if desired, as described above.

Veterinary intrammary injection

| | | mg/dose | Range |
|---|---|---|---|
| Active Ingredient | | 150mg | 0.05 - 1.0g |
| Polysorbate 60 | 3.0% w/w) | | ) |
| White Beeswax | 6.0% w/w) | to 3g | ) to 3 or 15g |
| Arachis oil | 91.0% w/w) | | ) |

Heat the arachis oil, white beeswax and polysorbate 60 to 160°C with stirring. Maintain at 160°C for two hours and then cool to room temperature with stirring. Aseptically add the active ingredient to the vehicle and disperse using a high speed mixer. Refine by passing through a colloid mill. Aseptically fill the product into sterile plastic syringes.

Veterinary oral drench

| | % w/v | Range |
|---|---|---|
| Active Ingredient | 0.35 | 0.01 - 2% w/v |
| Polysorbate 85 | 5.0 | |
| Benzyl alcohol | 3.0 | |
| Propylene glycol | 30.0 | |
| Phosphate buffer | as pH 6.0 - 6.5 | |
| Water | to 100.0 | |

Dissolve the active ingredient in the Polysorbate 85, benzyl alcohol and the propylene glycol. Add a proportion of the water and adjust the pH to 6.0 - 6.5 with phosphate buffer, if necessary. Make up to final volume with the water. Fill the product into the drench container.

Veterinary oral paste

| | % w/w | Range |
|---|---|---|
| Active Ingredient | 7.5 | 1 - 30% w/w |
| Saccharin | 25.0 | |
| Polysorbate 85 | 3.0 | |
| Aluminium distearate | 5.0 | |
| Fractionated coconut oil | to 100.0 | |

Disperse the aluminium distearate in the fractionated coconut oil and polysorbate 85 by heating. Cool to room temperature and disperse the saccharin in the oily vehicle. Dispense the active ingredient in the base. Fill into plastic syringes.

Granules for veterinary in-feed administration

| | % w/w | Range |
|---|---|---|
| Active Ingredient | 2.5 | 0.05-5% w/w |
| Calcium sulphate, hemi-hydrate | to 100.0 | |

Blend the Active Ingredient with the calcium sulphate. Prepare the granules using a wet granulation process. Dry using a tray or fluid-bed drier. Fill into the appropriate container.

Emulsifiable Concentrate

| | |
|---|---|
| Active ingredient | 50g |
| Anionic emulsifier (e.g. Phenyl sulphonate CALX) | 40g |
| Non-ionic emulsifier (e.g. Syperonic NP13) | 60g |
| Aromatic solvent (e.g. Solvesso 100) to 1 litre. | |

Mix all ingredients, stir until dissolved.

Granules

| (a) | Active ingredient<br>Wood resin | 50g<br>40g |
| --- | --- | --- |
| Gypsum granules (20-60 mesh) to 1kg (e.g. Agsorb 100A) | | |

| (b) | Active ingredient<br>Syperonic NP13 | 50g<br>40g |
| --- | --- | --- |
| Gypsum granules (20-60 mesh) to 1kg. | | |

Dissolve all ingredients in a volatile solvent e.g. methylene chloride, add to granules tumbling in mixer. Dry to remove solvent.

**Claims**

**Claims for the following Contracting States : BE, CH, DE, FR, GB, IT, LI, NL, SE**

**1.** Compounds of formula (I)

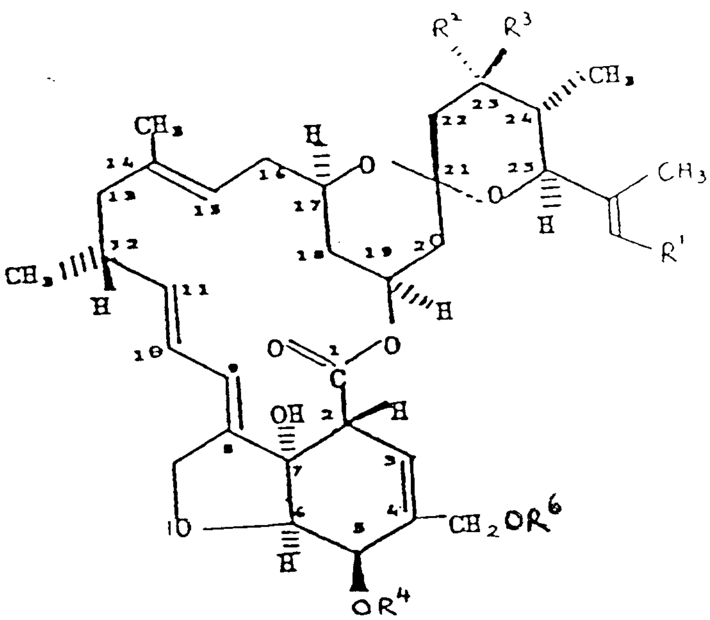

(I)

wherein

$R^1$ is an isopropyl group;

$R^2$ represents a hydrogen atom or a group $OR^5$ where $OR^5$ is a hydroxy group or a substituted hydroxyl which is a group $-OCOR^8$, $-OCO_2R^8$, where $R^8$ is $C_{1-8}$ alkyl; $C_{1-8}$ alkyl substituted by one or more halo, $C_{1-4}$ alkoxy, phenoxy or silyloxy substituents; $C_{2-8}$ alkenyl; $C_{2-8}$ alkynyl; $C_{3-12}$ cycloalkyl; phenylalkyl in which the alkyl portion has 1-6 carbon atoms; or phenyl; a formyloxy group, a group $-OR^9$ where $R^9$ is $C_{1-8}$ alkyl or $C_{1-8}$ alkyl substituted by $C_{3-7}$ cycloalkyl; a group $-OSO_2R^{10}$ where $R^{10}$ is a $C_{1-4}$ alkyl or toluyl; a silyloxy group; a tetrahydropyranyloxy group; a group $-OCO(CH_2)_nCO_2R^{11}$ where $R^{11}$ is a hydrogen atom or a $C_{1-4}$ alkyl group and n represents zero, 1 or 2; or a group $R^{12}R^{13}NCO_2$ where $R^{12}$ and $R^{13}$ are independently hydrogen atoms or $C_{1-4}$ alkyl groups; and

$R^3$ is a hydrogen atom, or $R^2$ and $R^3$ together with the carbon atom to which they are attached represent $>C=O$, $>C=CH_2$ or $>C=NOR^7$ where $R^7$ is a hydrogen atom, a $C_{1-8}$ alkyl group or a $C_{3-8}$ alkenyl group, and the group $>C=NOR^7$ is in the E configuration;

$OR^4$ represents a group $OR^5$ as defined above; and
$R^6$ is a hydrogen atom or a $C_{1-4}$ alkanoyl group.

2. Compounds according to claim 1 in which $R^2$ is a hydrogen atom or a hydroxy, $C_{1-8}$ alkoxy, cyclopropylmethoxy, cyclopropanecarbonyloxy or $C_{1-8}$ alkanoyloxy group and $R^3$ is a hydrogen atom, or $R^2$ and $R^3$ together with the carbon atom to which they are attached form a $>C=0$, $>C=CH_2$ or $>C=NOCH_3$ group.

3. Compounds according to any preceding claim in which $OR^4$ is a hydroxy, methoxy, acetoxy or methyloxycarbonyloxy group.

4. Compounds according to claim 1 in which $R^1$ is an isopropyl group, $R^2$ is a hydroxyl or acetoxy group, $R^3$ is a hydrogen atom, $OR^4$ is a hydroxy or acetoxy group and $R^6$ is a hydrogen atom or an acetyl group.

5. The compound according to claim 1 in which $R^1$ is an isopropyl group, $R^2$ and $R^3$ are hydrogen atoms, $OR^4$ is an acetoxy group and $R^6$ is a hydrogen atom.

6. A composition for use in human medicine containing an effective amount of at least one compound according to claim 1 together with one or more carriers and/or excipients.

7. A composition for use in veterinay medicine containing an effective amount of at least one compound according to claim 1 together with one or more carriers and/or excipients.

8. A pest control composition containing an effective amount of at least one compound according to claim 1 together with one or more carriers and/or excipients.

9. A method for combatting pests in agriculture, horticulture or forestry, or in stores, buildings or other public places or locations of the pests, which comprises applying to plants or other vegetation or to the pests themselves or a location thereof an effective amount of one or more compounds according to claim 1.

10. A method as claimed in claim 9 in which said pests are insect, acarine or nematode pests.

11. A process for the preparation of a compound according to claim 1 which comprises:
(a) in the preparation of a compound in which $R^6$ is a hydrogen atom, selectively oxidising the 4-methyl group of a compound formula (II)

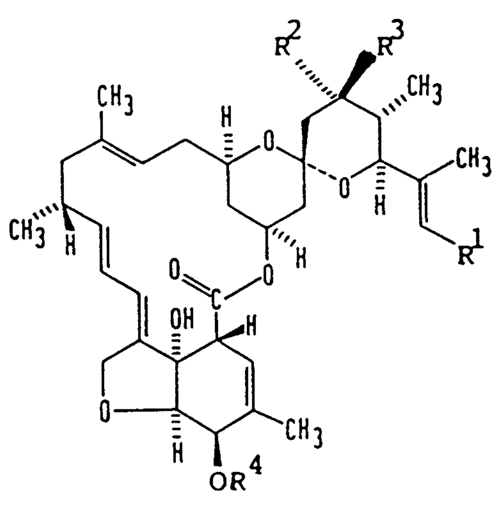

(II)

(b) in the preparation of a compound in which $R^6$ is a $C_{1-4}$ alkanoyl group, selectively acylating a corresponding compound in which $R^6$ is a hydrogen atom;

(c) in the preparation of a compound in which one of $R^2$ and $OR^4$ is a substituted hydroxy group and the other is a hydroxy or substituted hydroxy group and $R^6$ is a $C_{1-4}$ alkanoyl group, treating a corresponding compound (in which one of $R^2$ and $-OR^4$ is a hydroxyl group while the other is a hydroxy or substituted hydroxy group and $R^6$ is a hydrogen atom or a $C_{1-4}$ alkanoyl group) with a reagent serving to convert a hydroxy group into a substituted hydroxy group; and if desired followed by selective deprotection of a compound of formula (I) in which $R^2$ and $-OR^4$ are both substituted hydroxy groups to give a compound of formula (I) in which $OR^4$ is a hydroxy group and $R^2$ is a substituted hydroxy group; or

(d) in the preparation of a compound in which $R^2$ and/or $OR^4$ is hydroxy group, removing the protecting group from a corresponding compound in which $R^2$ and/or $OR^4$ is a protected hydroxy group.

**Claims for the following Contracting States : AT, ES**

**1.** A process for the preparation of compounds of formula (I)

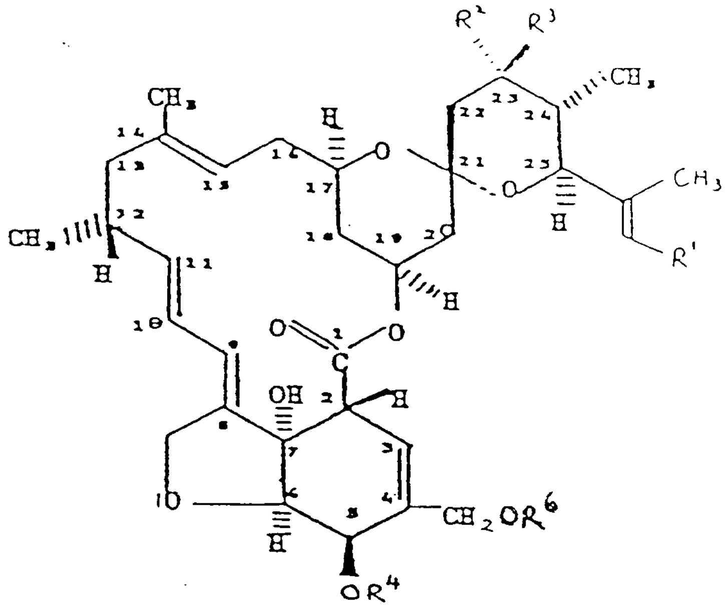

(I)

wherein

$R^1$ is an isopropyl group;

$R^2$ represents a hydrogen atom or a group $OR^5$ where $OR^5$ is a hydroxy group or a substituted hydroxyl which is a group $-OCOR^8$, $-OCO_2R^8$, wherein $R^8$ is $C_{1-8}$ alkyl; $C_{1-8}$ alkyl substituted by one or more halo, $C_{1-4}$ alkoxy, phenoxy or silyloxy substituents; $C_{2-8}$ alkenyl; $C_{2-8}$ alkynyl; $C_{3-12}$ cycloalkyl; phenylalkyl in which the alkyl portion has 1-6 carbon atoms; or phenyl; a formyloxy group, a group $-OR^9$ where $R^9$ is $C_{1-8}$ alkyl or $C_{1-8}$ alkyl substituted by $C_{3-7}$ cycloalkyl; a group $-OSO_2R^{10}$ where $R^{10}$ is a $C_{1-4}$ alkyl or toluyl; a silyloxy group; a tetrahydropyranyloxy group; a group $-OCO-(CH_2)_nCO_2R^{11}$ where $R^{11}$ is a hydrogen atom or a $C_{1-4}$ alkyl group and n represents zero, 1 or 2; or a group $R^{12}R^{13}NCO_2$ where $R^{12}$ and $R^{13}$ are independently hydrogen atoms or $C_{1-4}$ alkyl groups; and

$R^3$ is a hydrogen atom, or $R^2$ and $R^3$ together with the carbon atom to which they are attached represent $>C=O$, $>C=CH_2$ or $>C=NOR^7$ where $R^7$ is a hydrogen atom, a $C_{1-8}$ alkyl group or a $C_{3-8}$ alkenyl group, and the group $>C=NOR^7$ is in the E configuration;

$OR^4$ represents a group $OR^5$ as defined above; and

$R^6$ is a hydrogen atom or a $C_{1-4}$ alkanoyl group;

which process comprises:

(a) in the preparation of a compound in which $R^6$ is a hydrogen atom, selectively oxidising the 4-methyl group of a compound formula (II)

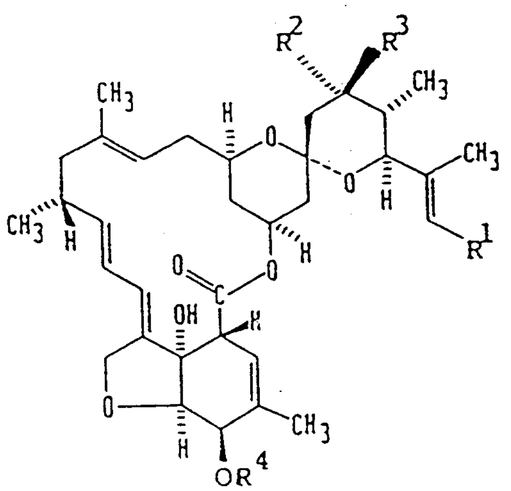

(II)

(b) in the preparation of a compound in which $R^6$ is a $C_{1-4}$ alkanoyl group, selectively acylating a corresponding compound in which $R^6$ is a hydrogen atom;

(c) in the preparation of a compound in which one of $R^2$ and $OR^4$ is a substituted hydroxy group and the other is a hydroxy or substituted hydroxy group and $R^6$ is a $C_{1-4}$ alkanoyl group, treating a corresponding compound (in which one of $R^2$ and $-OR^4$ is a hydroxyl group while the other is a hydroxy or substituted hydroxy group and $R^6$ is a hydrogen atom or a $C_{1-4}$ alkanoyl group) with a reagent serving to convert a hydroxy group into a substituted hydroxy group; and if desired followed by selective deprotection of a compound of formula (I) in which $R^2$ and $-OR^4$ are both substituted hydroxy groups to give a compound of formula (I) in which $OR^4$ is a hydroxy group and $R^2$ is a substituted hydroxy group; or

(d) in the preparation of a compound in which $R^2$ and/or $OR^4$ is hydroxy group, removing the protecting group from a corresponding compound in which $R^2$ and/or $OR^4$ is a protected hydroxy group.

**2.** A process according to claim 1 in which $R^2$ in the product is a hydrogen atom or a hydroxy, $C_{1-8}$ alkoxy, cyclopropylmethoxy, cyclopropanecarbonyloxy or $C_{1-8}$ alkanoyloxy group and $R^3$ is a hydrogen atom, or $R^2$ and $R^3$ together with the carbon atom to which they are attached form a $>C=O$, $>C=CH_2$ or $>C=NOCH_3$ group.

**3.** A process according to any preceding claim in which $OR^4$ in the product is a hydroxy, methoxy, acetoxy or methyloxycarbonyloxy group.

**4.** A process according to claim 1 in which $R^1$ in the product is an isopropyl group, $R^2$ is a hydroxyl or acetoxy group, $R^3$ is a hydrogen atom, $OR^4$ is a hydroxy or acetoxy group and $R^6$ is a hydrogen atom or an acetyl group.

**5.** A process according to claim 1 in which, in the product, $R^1$ is an isopropyl group, $R^2$ and $R^3$ are hydrogen atoms, $OR^4$ is an acetoxy group and $R^6$ is a hydrogen atom.

**6.** A method for combatting pests in agriculture, horticulture or forestry, or in stores, buildings or other public places or locations of the pests, which comprises applying to plants or other vegetation or to the pests themselves or a location thereof an effective amount of one or more compounds of formula (I) as defined in claim 1.

**7.** A method as claimed in claim 6 in which said pests are insect, acarine or nematode pests.

**Patentansprüche**
**Patentansprüche für folgende Vertragsstaaten : BE, CH, DE, FR, GB, IT, LI, NL, SE**

1. Verbindungen der Formel (I)

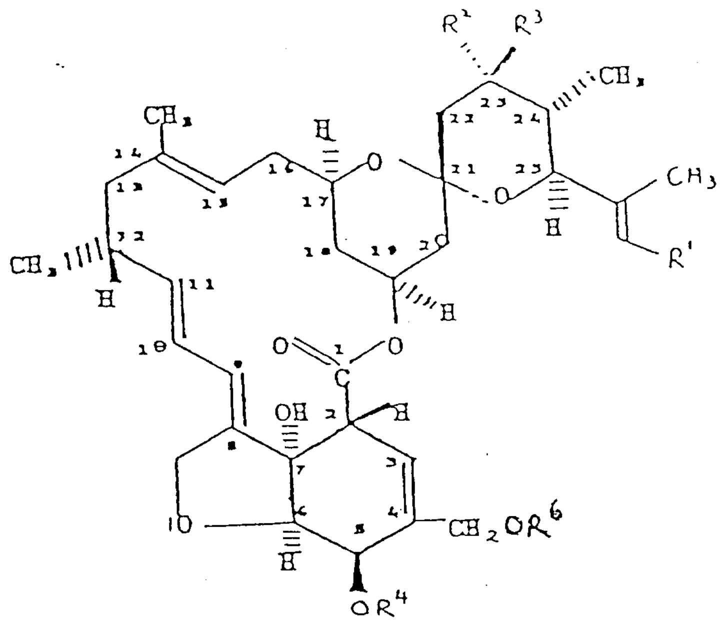

(I)

worin
$R^1$ für eine Isopropylgruppe steht;
$R^2$ ein Wasserstoffatom oder eine Gruppe $OR^5$, worin $OR^5$ eine Hydroxygruppe oder eine substituierte Hydroxylgruppe, die eine Gruppe $-OCOR^8$, $-OCO_2R^8$ ist, worin $R^8$ für $C_{1-8}$-Alkyl steht, bedeutet; $C_{1-8}$-Alkyl, substituiert durch einen oder mehrere Halo-, $C_{1-4}$-Alkoxy-, Phenoxy- oder Silyloxysubstituenten; $C_{2-8}$-Alkenyl; $C_{2-8}$-Alkinyl; $C_{3-12}$-Cycloalkyl; Phenylalkyl, worin der Alkylteil 1 bis 6 Kohlenstoffatome aufweist; oder Phenyl; eine Formyloxygruppe, eine Gruppe $-OR^9$, worin $R^9$ $C_{1-8}$-Alkyl oder $C_{1-8}$-Alkyl, substituiert durch $C_{3-7}$-Cycloalkyl, bedeutet; eine Gruppe $-OSO_2R^{10}$, worin $R^{10}$ $C_{1-4}$-Alkyl oder Toluyl ist; eine Silyloxygruppe; eine Tetrahydropyranyloxygruppe; eine Gruppe $-OCO(CH_2)_nCO_2R^{11}$, worin $R^{11}$ ein Wasserstoffatom oder eine $C_{1-4}$-Alkylgruppe bedeutet und n für 0, 1 oder 2 steht; oder eine Gruppe $R^{12}R^{13}NCO_2$, worin $R^{12}$ und $R^{13}$ unabhängig Wasserstoffatome oder $C_{1-4}$-Alkylgruppen darstellen, wiedergibt; und
$R^3$ ein Wasserstoffatom darstellt oder $R^2$ und $R^3$ gemeinsam mit dem Kohlenstoffatom, an das sie gebunden sind, für $>C=O$, $>C=CH_2$ oder $>C=NOR^7$ stehen, worin $R^7$ ein Wasserstoffatom, eine $C_{1-8}$-Alkylgruppe oder eine $C_{3-8}$-Alkenylgruppe bedeutet und sich die Gruppe $>C=NOR^7$ in der E-Konfiguration befindet;
$OR^4$ für eine Gruppe $OR^5$, wie vorstehend definiert, steht; und $R^6$ ein Wasserstoffatom oder eine $C_{1-4}$-Alkanoylgruppe bedeutet.

2. Verbindungen gemäß Anspruch 1, worin $R^2$ ein Wasserstoffatom oder eine Hydroxy-, $C_{1-8}$-Alkoxy-, Cyclopropylmethoxy-, Cyclopropancarbonyloxy- oder $C_{1-8}$-Alkanoyloxygruppe bedeutet und $R^3$ für ein Wasserstoffatom steht oder $R^2$ und $R^3$ gemeinsam mit dem Kohlenstoffatom, an das sie gebunden sind, eine $>C=O$-, $>C=CH_2$- oder $>C=NOCH_3$-Gruppe bilden.

3. Verbindungen gemäß einem der vorhergehenden Ansprüche, worin $OR^4$ eine Hydroxy-, Methoxy-, Acetoxy- oder Methyloxycarbonyloxygruppe bedeutet.

4. Verbindungen gemäß Anspruch 1, worin $R^1$ eine Isopropylgruppe darstellt, $R^2$ eine Hydroxyl- oder Acetoxygruppe bedeutet, $R^3$ für ein Wasserstoffatom steht, $OR^4$ eine Hydroxy- oder Acetoxygruppe ist und $R^6$ ein Wasserstoffatom oder eine Acetylgruppe wiedergibt.

**5.** Verbindungen gemäß Anspruch 1, worin $R^1$ eine Isopropylgruppe bedeutet, $R^2$ und $R^3$ Wasserstoffatome sind, $OR^4$ eine Acetoxygruppe wiedergibt und $R^6$ ein Wasserstoffatom ist.

**6.** Zusammensetzung für die Verwendung in der Humanmedizin, die eine wirksame Menge zumindest einer Verbindung gemäß Anspruch 1 zusammen mit einem oder mehreren Trägern und/oder Exzipienten enthält.

**7.** Zusammensetzung für die Verwendung in der Veterinärmedizin, die eine wirksame Menge zumindest einer Verbindung gemäß Anspruch 1 zusammen mit einem oder mehreren Trägern und/oder Exzipienten enthält.

**8.** Zusammensetzung für die Schädlingskontrolle, die eine wirksame Menge zumindest einer Verbindung gemäß Anspruch 1 zusammen mit einem oder mehreren Trägern und/oder Exzipienten enthält.

**9.** Verfahren zur Bekämpfung von Schädlingen in der Landwirtschaft, im Gartenbau oder der Forstwirtschaft oder in Vorratslagern, Gebäuden oder an anderen öffentlichen Plätzen oder Aufenthaltsorten der Schädlinge, das das Aufbringen einer wirksamen Menge einer oder mehrerer Verbindungen gemäß Anspruch 1 auf Pflanzen oder andere Vegetation oder auf die Schädlinge selbst oder einen Aufenthaltsort derselben umfaßt.

**10.** Verfahren gemäß Anspruch 9, worin die Schädlinge Insekten, Milben oder Nematodenschädlinge sind.

**11.** Verfahren zur Herstellung einer Verbindung gemäß Anspruch 1, das umfaßt:

(a) bei der Herstellung einer Verbindung, worin $R^6$ ein Wasserstoffatom ist, die selektive Oxidation der 4-Methylgruppe einer Verbindung der Formel (II)

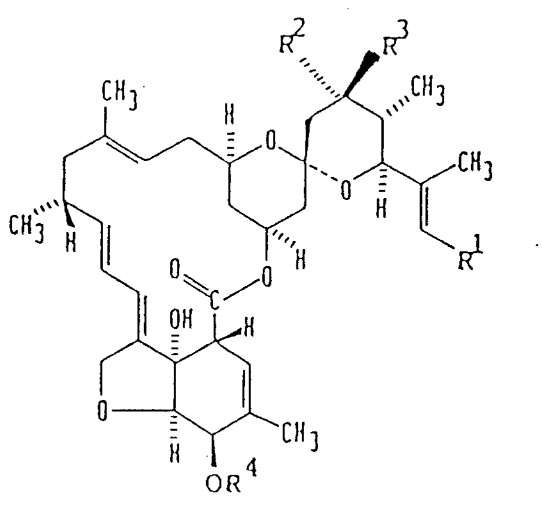

(II)

(b) bei der Herstellung einer Verbindung, worin $R^6$ für eine $C_{1-4}$-Alkanoylgruppe steht, die selektive Acylierung einer entsprechenden Verbindung, worin $R^6$ ein Wasserstoffatom ist;

(c) bei der Herstellung einer Verbindung, worin eines von $R^2$ und $OR^4$ eine substituierte Hydroxygruppe bedeutet und das andere eine Hydroxy- oder substituierte Hydroxygruppe ist und $R^6$ eine $C_{1-4}$-Alkanoylgruppe darstellt, die Behandlung einer entsprechenden Verbindung (worin eines von $R^2$ und $-OR^4$ eine Hydroxygruppe ist, während das andere eine Hydroxy- oder substituierte Hydroxygruppe bedeutet und $R^6$ für ein Wasserstoffatom oder eine $C_{1-4}$-Alkanoylgruppe steht) mit einem Reagens, das der Umwandlung einer Hydroxygruppe in eine substituierte Hydroxygruppe dient, und gegebenenfalls die anschließende selektive Schutzgruppenabspaltung von einer Verbindung der Formel (I), worin $R^2$ und $-OR^4$ beide substituierte Hydroxygruppen darstellen, um eine Verbindung der Formel (I) zu ergeben, worin $OR^4$ eine Hydroxygruppe bedeutet und $R^2$ eine substituierte Hydroxygruppe ist; oder

(d) bei der Herstellung einer Verbindung, worin $R^2$ und/oder $OR^4$ eine Hydroxygruppe bedeutet, die Entfernung der Schutzgruppe aus einer entsprechenden Verbindung, worin $R^2$ und/oder $OR^4$ eine geschützte Hydroxygruppe ist.

**Patentansprüche für folgende Vertragsstaaten : AT, ES**

1. Verfahren zur Herstellung von Verbindungen der Formel (I)

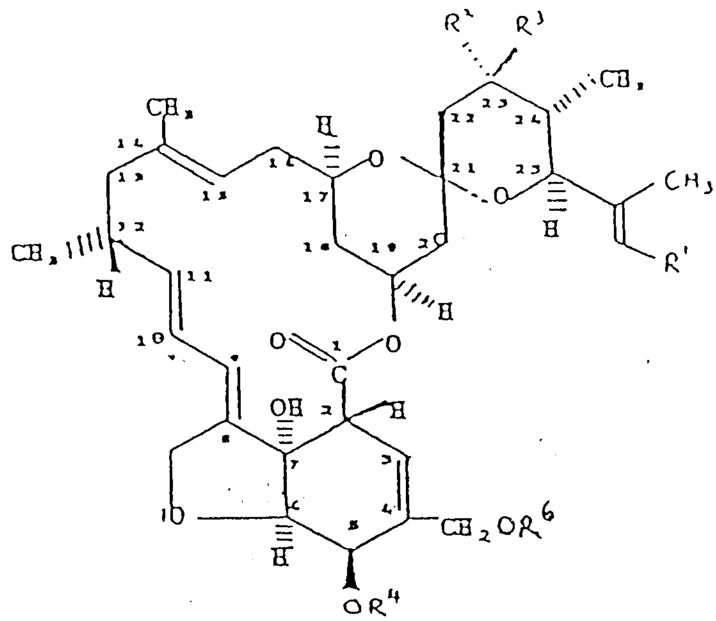

(I)

worin

$R^1$ für eine Isopropylgruppe steht;

$R^2$ ein Wasserstoffatom oder eine Gruppe $OR^5$, worin $OR^5$ eine Hydroxygruppe oder eine substituierte Hydroxylgruppe, die eine Gruppe $-OCOR^8$, $-OCO_2R^8$ ist, worin $R^8$ für $C_{1-8}$-Alkyl steht, bedeutet; $C_{1-8}$-Alkyl, substituiert durch einen oder mehrere Halo-, $C_{1-4}$- Alkoxy-, Phenoxy-oder Silyloxysubstituenten; $C_{2-8}$-Alkenyl; $C_{2-8}$-Alkinyl; $C_{3-12}$-Cycloalkyl; Phenylalkyl, worin der Alkylteil 1 bis 6 Kohlenstoffatome aufweist; oder Phenyl; eine Formyloxygruppe, eine Gruppe $-OR^9$, worin $R^9$ $C_{1-8}$-Alkyl oder $C_{1-8}$-Alkyl, substituiert durch $C_{3-7}$-Cycloalkyl, bedeutet; eine Gruppe $-OSO_2R^{10}$, worin $R^{10}$ $C_{1-4}$-Alkyl oder Toluyl ist; eine Silyloxygruppe; eine Tetrahydropyranyloxygruppe; eine Gruppe $-OCO(CH_2)_nCO_2R^{11}$, worin $R^{11}$ ein Wasserstoffatom oder eine $C_{1-4}$-Alkylgruppe bedeutet und n für 0, 1 oder 2 steht; oder eine Gruppe $R^{12}R^{13}NCO_2$, worin $R^{12}$ und $R^{13}$ unabhängig Wasserstoffatome oder $C_{1-4}$-Alkylgruppen darstellen, wiedergibt; und

$R^3$ ein Wasserstoffatom darstellt oder $R^2$ und $R^3$ gemeinsam mit dem Kohlenstoffatom, an das sie gebunden sind, für $>C=O$, $>C=CH_2$ oder $>C=NOR^7$ stehen, worin $R^7$ ein Wasserstoffatom, eine $C_{1-8}$-Alkylgruppe oder eine $C_{3-8}$-Alkenylgruppe bedeutet und sich die Gruppe $>C=NOR^7$ in der E-Konfiguration befindet;

$OR^4$ für eine Gruppe $OR^5$, wie vorstehend definiert, steht; und $R^6$ ein Wasserstoffatom oder eine $C_{1-4}$-Alkanoylgruppe bedeutet;

wobei das Verfahren umfaßt:

(a) bei der Herstellung einer Verbindung, worin $R^6$ ein Wasserstoffatom ist, die selektive Oxidation der 4-Methylgruppe einer Verbindung der Formel (II)

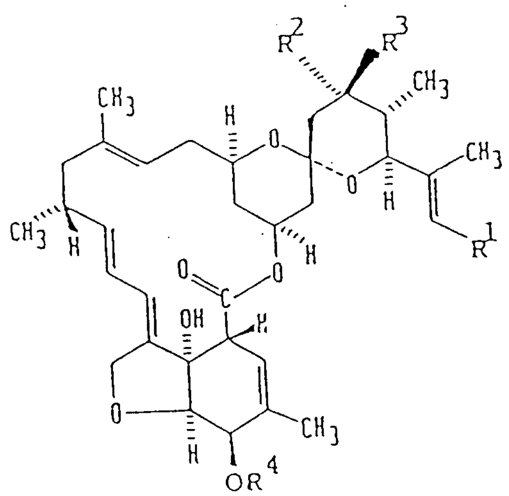

(II)

(b) bei der Herstellung einer Verbindung, worin $R^6$ für eine $C_{1-4}$-Alkanoylgruppe steht, die selektive Acylierung einer entsprechenden Verbindung, worin $R^6$ ein Wasserstoffatom ist;
(c) bei der Herstellung einer Verbindung, worin eines von $R^2$ und $OR^4$ eine substituierte Hydroxygruppe bedeutet und das andere eine Hydroxy- oder substituierte Hydroxygruppe ist und $R^6$ eine $C_{1-4}$-Alkanoylgruppe darstellt, die Behandlung einer entsprechenden Verbindung (worin eines von $R^2$ und $-OR^4$ eine Hydroxygruppe ist, während das andere eine Hydroxy- oder substituierte Hydroxygruppe bedeutet und $R^6$ für ein Wasserstoffatom oder eine $C_{1-4}$-Alkanoylgruppe steht) mit einem Reagens, das der Umwandlung einer Hydroxygruppe in eine substituierte Hydroxygruppe dient, und gegebenenfalls die anschließende selektive Schutzgruppenabspaltung von einer Verbindung der Formel (I), worin $R^2$ und $-OR^4$ beide substituierte Hydroxygruppen darstellen, um eine Verbindung der Formel (I) zu ergeben, worin $OR^4$ eine Hydroxygruppe bedeutet und $R^2$ eine substituierte Hydroxygruppe ist; oder
(d) bei der Herstellung einer Verbindung, worin $R^2$ und/oder $OR^4$ eine Hydroxygruppe bedeutet, die Entfernung der Schutzgruppe aus einer entsprechenden Verbindung, worin $R^2$ und/oder $OR^4$ eine geschützte Hydroxygruppe ist.

2. Verfahren gemäß Anspruch 1, worin $R^2$ in dem Produkt ein Wasserstoffatom oder eine Hydroxy-, $C_{1-8}$-Alkoxy-, Cyclopropylmethoxy-, Cyclopropancarbonyloxy- oder $C_{1-8}$-Alkanoyloxygruppe bedeutet und $R^3$ für ein Wasserstoffatom steht oder $R^2$ und $R^3$ gemeinsam mit dem Kohlenstoffatom, an das sie gebunden sind, eine $>C=O$-, $>C=CH_2$- oder $>C=NOCH_3$-Gruppe bilden.

3. Verfahren gemäß einem der vorhergehenden Ansprüche, worin $OR^4$ in dem Produkt eine Hydroxy-, Methoxy-, Acetoxy- oder Methyloxycarbonyloxygruppe bedeutet.

4. Verfahren gemäß Anspruch 1, worin $R^1$ in dem Produkt eine Isopropylgruppe darstellt, $R^2$ eine Hydroxyl- oder Acetoxygruppe bedeutet, $R^3$ für ein Wasserstoffatom steht, $OR^4$ eine Hydroxy- oder Acetoxygruppe ist und $R^6$ ein Wasserstoffatom oder eine Acetylgruppe bedeutet.

5. Verfahren gemäß Anspruch 1, worin in dem Produkt $R^1$ eine Isopropylgruppe bedeutet, $R^2$ und $R^3$ Wasserstoffatome sind, $OR^4$ eine Acetoxygruppe wiedergibt und $R^6$ ein Wasserstoffatom ist.

6. Verfahren zur Bekämpfung von Schädlingen in der Landwirtschaft, dem Gartenbau oder der Forstwirtschaft oder in Vorratslagern, Gebäuden oder anderen öffentlichen Plätzen oder Aufenthaltsorten von Schädlingen, das das Aufbringen einer wirksamen Menge einer oder mehrerer Verbindungen der Formel (I), wie in Anspruch 1 definiert, auf Pflanzen oder andere Vegetation oder auf die Schädlinge selbst oder einem Aufenthaltsort desselben umfaßt.

7. Verfahren gemäß Anspruch 6, worin die Schädlinge Insekten, Milben oder Nematodenschädlinge sind.

**Revendications**

**Revendications pour les Etats contractants suivants : BE, CH, DE, FR, GB, IT, LI, NL, SE**

1. Composés de formule I

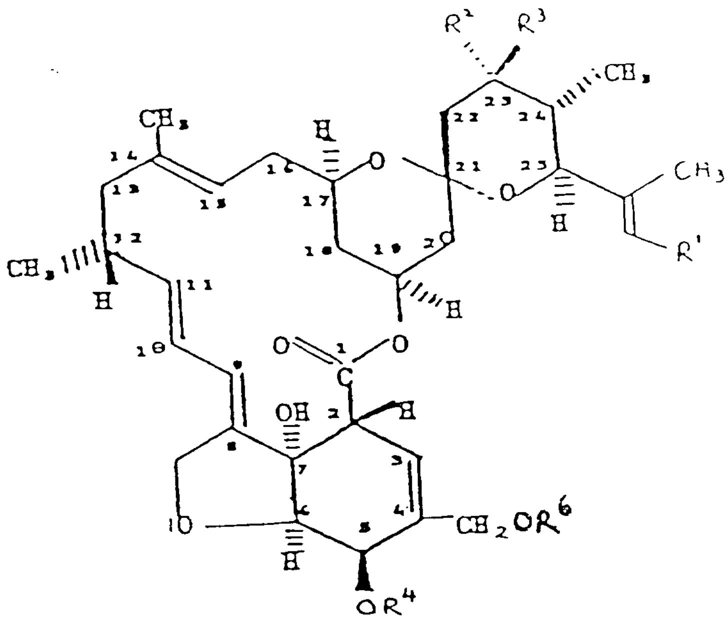

(I)

dans laquelle
$R^1$ est un groupe isopropyle;
$R^2$ représente un atome d'hydrogène ou un groupe $-OR^5$ où $R^5$ est un groupe hydroxyle ou un groupe hydroxyle substitué qui est un groupe $-OCOR^8$, $-OCO_2R^8$ où $R^8$ est un $(C_1-C_8)$alkyle, un $(C_1-C_8)$alkyle substitué par un ou plusieurs halogène, $(C_1-C_4)$alcoxy, phénoxy ou silyloxy; $(C_2-C_8)$alcényle; $(C_2-C_8)$-alcynyle; $(C_3-C_{12})$cycloalkyle; phénylalkyle dans lequel la partie alkyle contient de 1 à 6 atomes de carbone; ou phényle; un groupe formyloxy, un groupe $-OR^9$ où $R^9$ est un $(C_1-C_8)$alkyle ou un $(C_1-C_8)$-alkyle substitué par un $(C_3-C_7)$cycloalkyle; un groupe $-OSO_2R^{10}$ où $R^{10}$ est un $(C_1-C_4)$alkyle ou toluyl; un groupe silyloxy; un groupe tétrahydropyranyloxy; un groupe $-OCO(CH_2)_nCO_2R^{11}$ où $R^{11}$ est un atome d'hydrogène ou un groupe $(C_1-C_4)$alkyle et n représente 0, 1 ou 2; ou un groupe $R^{12}R^{13}NCO_2$ où $R^{12}$ et $R^{13}$ sont indépendamment des atomes d'hydrogène ou des groupes $(C_1-C_4)$alkyles; et $R^3$ est un atome d'hydrogène, ou $R^2$ et $R^3$ pris ensemble avec l'atome de carbone auquel ils sont attachés représentent $>C=O$, $>C=CH_2$ ou $>C=N-OR^7$ où $R^7$ est un atome d'hydrogène, un groupe $(C_1-C_8)$alkyle ou un groupe $(C_3-C_8)$alcényle, et le groupe $>C=N-OR^7$ est dans la configuration E;
$OR^4$ représente un groupe $OR^5$ tel que défini ci-dessus; et $R^6$ est un atome d'hydrogène ou un groupe $(C_1-C_4)$alcanoyle.

2. Composés selon la revendication 1, dans lesquels $R^2$ est un atome d'hydrogène ou un hydroxy, $(C_1-C_8)$alcoxy, cyclopropylmethoxy, cyclopropanecarbonyloxy ou un groupe $(C_1-C_8)$alcanoyloxy et $R^3$ est un atome d'hydrogène, ou $R^2$ et $R^3$ pris ensemble avec la carbone auquel ils sont attachés forment un groupe $>C=O$, $>C=CH_2$ ou $>C=N-OCH_3$.

3. Composés selon l'une quelconque des revendications précédentes, dans lesquels $OR^4$ est un groupe hydroxy, méthoxy, acétoxy ou méthyloxycarbonyloxy.

4. Composés selon la revendication 1, dans lesquels $R^1$ est un groupe isopropyle, $R^2$ est un groupe hydroxyle ou acétoxy, $R^3$ est un atome d'hydrogène, $OR^4$ est un groupe hydroxy ou acétoxy et $R^6$ est un atome d'hydrogène ou un groupe acétyle.

5. Composé selon la revendication 1, dans lequel $R^1$ est un groupe isopropyle, $R^2$ et $R^3$ sont des atomes d'hydrogène, $OR^4$ est un groupe acétoxy et $R^6$ est un atome d'hydrogène.

**6.** Composition destinée à être utilisée en médecine humaine contenant une quantité efficace d'au moins un composé selon la revendication 1 en association avec un ou plusieurs véhicules et/ou excipients.

**7.** Composition destinée à être utilisée en médecine animale contenant une quantité efficace d'au moins un composé selon la revendication 1 en association avec un ou plusieurs véhicules et/ou excipients.

**8.** Composition pour l'élimination des animaux nuisibles contenant une quantité efficace d'au moins un composé selon la revendication 1 en association avec un ou plusieurs véhicules et/ou excipients.

**9.** Procédé pour lutter contre les animaux nuisibles dans l'agriculture, l'horticulture et la sylviculture ou dans les magasins, immeubles ou autres lieux publics ou habitats des animaux nuisibles, qui consiste à appliquer sur les plantes ou autre végétation ou bien sur les animaux nuisibles eux-mêmes ou sur leur habitat une quantité efficace d'un ou plusieurs composés selon la revendication 1.

**10.** Procédé tel que revendiqué à la revendication 9 dans lequel lesdits animaux nuisibles sont des insectes nuisibles, des acariens ou des nématodes nuisibles.

**11.** Procédé de préparation d'un composé selon la revendication 1 qui comprend :

(a) pour la préparation d'un composé pour lequel $R^6$ est un atome d'hydrogène, l'oxydation sélective du groupe méthyle en 4 d'un composé de formule II

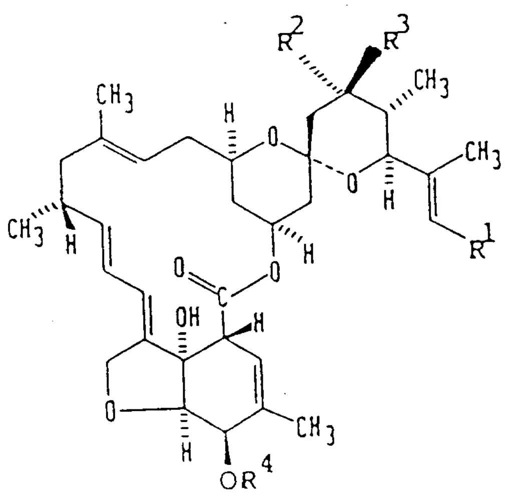

(II)

(b) pour la préparation d'un composé pour lequel $R^6$ est un groupe ($C_1$-$C_4$)alcanoyle, l'acylation sélective du composé correspondant pour lequel $R^6$ est un atome d'hydrogène.

(c) pour la préparation d'un composé pour lequel l'un des groupes $R^2$ et -$OR^4$ est un groupe hydroxyle substitué et l'autre est un groupe hydroxyle ou un groupe hydroxyle substitué et $R^6$ est un groupe ($C_1$-$C_4$)alcanoyle, le traitement du composé correspondant (pour lequel l'un des groupes $R^2$ et -$OR^4$ est un groupe hydroxyle alors que l'autre est un groupe hydroxyle ou un groupe hydroxyle substitué et $R^6$ est un atome d'hydrogène ou un groupe ($C_1$-$C_4$)alcanoyle) par un réactif permettant la conversion du groupe hydroxyle en groupe hydroxyle substitué; et le cas échéant suivi de la déprotection sélective du composé de formule (I) dans laquelle $R^2$ et -$OR^4$ sont tous les deux des groupes hydroxyle substitués conduisant à un composé de formule (I) dans laquelle -$OR^4$ est un groupe hydroxyle et $R^2$ est un groupe hydroxyle substitué; ou

(d) pour la préparation d'un composé dans lequel $R^2$ et/ou $OR^4$ est un groupe hydroxyle, l'élimination du groupe protecteur à partir du composé correspondant pour lequel $R^2$ et/ou $OR^4$ est un groupe hydroxyle protégé.

**Revendications pour les Etats contractants suivants : AT, ES**

**1.** Procédé pour la préparation de composés de formule (I)

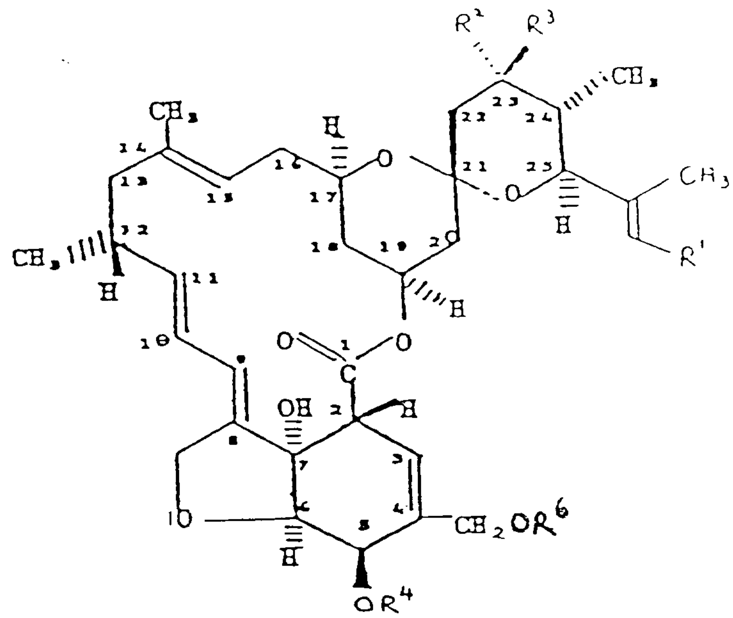

(I)

dans laquelle
$R^1$ est un groupe isopropyle;
$R^2$ représente un atome d'hydrogène ou un groupe $-OR^5$ où $R^5$ est un groupe hydroxyle ou un groupe hydroxyle substitué qui est un groupe $-OCOR^8$, $-OCO_2R^8$ où $R^8$ est un $(C_1-C_8)$alkyle, un $(C_1-C_8)$alkyle substitué par un ou plusieurs halogène, $(C_1-C_4)$alcoxy, phénoxy ou silyloxy; $(C_2-C_8)$alcényle; $(C_2-C_8)$-alcynyle; $(C_3-C_{12})$cycloalkyle; phénylalkyle dans lequel la partie alkyle contient de 1 à 6 atomes de carbone; ou phényle; un groupe formyloxy, un groupe $-OR^9$ où $R^9$ est un $(C_1-C_8)$alkyle ou un $(C_1-C_8)$-alkyle substitué par un $(C_3-C_7)$cycloalkyle; un groupe $-OSO_2R^{10}$ où $R^{10}$ est un $(C_1-C_4)$alkyle ou toluyl; un groupe silyloxy; un groupe tétrahydropyranyloxy; un groupe $-OCO(CH_2)_nCO_2R^{11}$ où $R^{11}$ est un atome d'hydrogène ou un groupe $(C_1-C_4)$alkyle et n représente 0, 1 ou 2; ou un groupe $R^{12}R^{13}NCO_2$ où $R^{12}$ et $R^{13}$ sont indépendamment des atomes d'hydrogène ou des groupes $(C_1-C_4)$alkyles; et $R^3$ est un atome d'hydrogène, ou $R^2$ et $R^3$ pris ensemble avec l'atome de carbone auquel ils sont attachés représentent $>C=O$, $>C=CH_2$ ou $>C=N-OR^7$ où $R^7$ est un atome d'hydrogène, un groupe $(C_1-C_8)$alkyle ou un groupe $(C_3-C_8)$alcényle, et le groupe $>C=N-OR^7$ est dans la configuration E;
$OR^4$ représente un groupe $OR^5$ tel que défini ci-dessus; et $R^6$ est un atome d'hydrogène ou un groupe $(C_1-C_4)$alcanoyle. lequel procédé comprend :

(a) pour la préparation d'un composé pour lequel $R^6$ est un atome d'hydrogène, l'oxydation sélective du groupe méthyle en 4 d'un composé de formule II

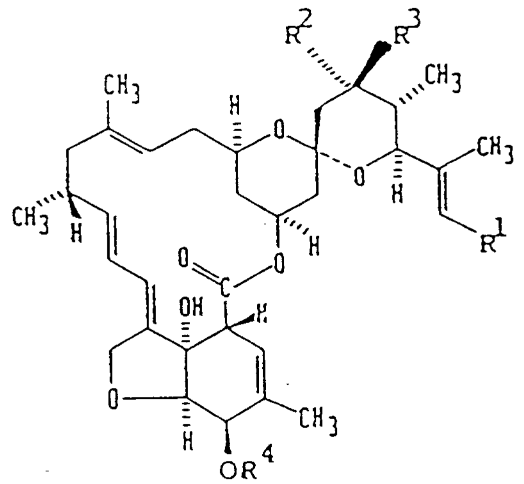

(II)

(b) pour la préparation d'un composé pour lequel $R^6$ est un groupe $(C_1\text{-}C_4)$alcanoyle, l'acylation sélective du composé correspondant pour lequel $R^6$ est un atome d'hydrogène.

(c) pour la préparation d'un composé pour lequel l'un des groupes $R^2$ et $-OR^4$ est un groupe hydroxyle substitué et l'autre est un groupe hydroxyle ou un groupe hydroxyle substitué et $R^6$ est un groupe $(C_1\text{-}C_4)$alcanoyle, le traitement du composé correspondant (pour lequel l'un des groupes $R^2$ et $-OR^4$ est un groupe hydroxyle alors que l'autre est un groupe hydroxyle ou un groupe hydroxyle substitué et $R^6$ est un atome d'hydrogène ou un groupe $(C_1\text{-}C_4)$alcanoyle) par un réactif permettant la conversion du groupe hydroxyle en groupe hydroxyle substitué; et le cas échéant suivi de la déprotection sélective du composé de formule (I) dans laquelle $R^2$ et $-OR^4$ sont tous les deux des groupes hydroxyle substitués conduisant à un composé de formule (I) dans laquelle $-OR^4$ est un groupe hydroxyle et $R^2$ est un groupe hydroxyle substitué; ou

(d) pour la préparation d'un composé dans lequel $R^2$ et/ou $OR^4$ est un groupe hydroxyle, l'élimination du groupe protecteur à partir du composé correspondant pour lequel $R^2$ et/ou $OR^4$ est un groupe hydroxyle protégé.

**2.** Procédé selon la revendication 1 dans lequel le produit est tel que $R^2$ est un atome d'hydrogène ou un hydroxyle, $(C_1\text{-}C_8)$alcoxy, cyclopropylmethoxy, cyclopropanecarbonyloxy ou un groupe $(C_1\text{-}C_8)$-alcanoyloxyet $R_3$ est un atome d'hydrogène, ou $R^2$ et $R^3$ pris ensemble avec la carbone auquel ils sont attachés forment un groupe $>C=O$, $>C=CH_2$ ou $>C=N\text{-}OCH_3$.

**3.** Procédé selon l'une quelconque des revendications précédentes dans lequel le procédé est tel que $-OR^4$ est un groupe hydroxy, méthoxy, acétoxy ou méthyloxycarbonyloxy.

**4.** Procédé selon la revendication 1 dans lequel le procédé est tel que $R^1$ est un groupe isopropyle, $R^2$ est un groupe hydroxyle ou acétoxy, $R^3$ est un atome d'hydrogène, $OR^4$ est un groupe hydroxy ou acétoxy et $R^6$ est un atome d'hydrogène ou un groupe acétyle.

**5.** Procédé selon la revendication 1 dans lequel le procédé est tel que $R^1$ est un groupe isopropyle, $R^2$ et $R^3$ sont des atomes d'hydrogène, $OR^4$ est un groupe acétoxy et $R^6$ est un atome d'hydrogène.

**6.** Procédé pour lutter contre les animaux nuisibles dans l'agriculture, l'horticulture et la sylviculture ou dans les magasins, immeubles ou autres lieux publics ou habitats des animaux nuisibles, qui consiste à appliquer sur les plantes ou autre végétation ou bien sur les animaux nuisibles eux-mêmes ou sur leur habitat une quantité efficace d'un ou plusieurs composés de formule (I) telle que définie dans la revendication 1.

**7.** Procédé tel que revendiqué à la revendication 6 dans lequel lesdits animaux nuisibles sont des insectes nuisibles, des acariens ou des nématodes nuisibles.